# EUROPEAN PATENT APPLICATION

(11) **EP 4 831 675 A1**
(43) Date of publication of application: **28.10.2026**
(21) Application number: 25172596.6
(22) Date of filing: 25.04.2025
(51) Int. Cl.: G01R 33/565, A61B 5/055, G01R 33/567

(54) **METHOD AND SYSTEM FOR REAL-TIME BULK-MOTION DETECTION DURING MRI**

(71) Applicant: Siemens Healthineers AG, 91301 Forchheim (DE); Centre Hospitalier Universitaire Vaudois, 1011 Lausanne (CH)
(72) Inventor: Müller, Marco, 1022 Chavannes-près-Renens (CH); Romanin, Ludovica, 1005 Lausanne (CH); Roy, Christopher, 1012 Lausanne (CH); Stuber, Matthias, 1032 Romanel-sur- Lausanne (CH); Hilbert, Tom, 1006 Lausanne (CH)
(74) Representative: Fischer, Michael

(57) **Abstract**

The invention relates to a method and system for real-time bulk-motion detection of a biological object (110) during magnetic resonance imaging, hereafter MRI, of an internal part (111) of said biological object (110), wherein said internal part (111) is characterized by a repeated motion over time, said method (200) comprising:
- launching (201) an acquisition stage (500), wherein during said acquisition stage (500), an MRI signal that enables an image reconstruction of said internal part (111) and a tracker signal modulated by said repeated motion of said internal part (111) of the biological object (110) are acquired;
characterized in that the method comprises
- using (202) the tracker signal acquired during a reference stage (510) for automatically extracting or determining, from said tracker signal, a reference pattern (410) of said repeated motion, wherein said reference stage (510) is comprised within said acquisition stage (500), takes place at the beginning of said acquisition stage (500), and extends over a time period Tref covering at least one repetition of said repeated motion;
- after the end of said time period Tref, using (203) said reference pattern (410) for automatically detecting, in real time and from the currently acquired tracker signal, a bulk-motion of the biological object (110); and
- outputting (204) an alert in function of the detected bulk-motion.

## Description

### Technical Field

The present invention proposes a system and a method for real-time bulk-motion detection of a biological object during Magnetic Resonance Imaging (MRI). More generally speaking, it concerns MRI techniques used for imaging or scanning a biological object, like tissue, and more specifically techniques for imaging a biological object comprising an internal moving part, like the heart.

### Background Art

It is known that motion and motion-related artifacts need to be addressed when imaging moving organs, such as the heart or the liver, with a relatively slow acquisition technique such as MRI. Occasionally, the problematic of object motion during imaging is present also for structures that do not intrinsically move, such as the brain, when for instance a patient cannot lay still in an MRI scanner for the whole duration of the image acquisition. In general, "motion handling" techniques are either directed to trigger or gate the acquisition to a quiescent motion period (e.g. mid diastole or end-expiration), or to extract motion information and use it e.g. for rejecting or correcting the motion-corrupted data. The identification of motion is usually based on techniques that make prior assumptions about the physiological processes that give rise to the motion signals. Such motion signals can be either extracted from imaging data, e.g. the MRI data itself using so-called image navigators, or from external sources as the ECG or a respiratory belt (or e.g. a camera, when it comes to head motion).

However, non-periodical motion or random motion events, which are referred to as *bulk-motion,* cannot be accounted for by such techniques. Bulk-motion refers to large, involuntary movements of the imaged biological object during image acquisition, e.g. motion of a patient's body during an MRI scan. Unlike small, controlled movements like breathing or heartbeat (which can be accounted for with specialized techniques), bulk-motion includes sudden shifts such as a patient adjusting position, coughing, or involuntarily twitching. This type of movement is particularly problematic in the field of MRI, because MRI scans rely on capturing data over an extended period of time, usually several minutes, and any unexpected movement can lead to the following negative effects:
- reduced Image Quality: MRI works by collecting signals over time to construct a detailed image. If the patient moves during the scan, the signals no longer align correctly, leading to blurring, ghosting, or artifacts (unwanted distortions). This can make the images unreadable or at least significantly harder to interpret;
- inefficient Diagnosis: If a scan is affected by bulk-motion, the radiologist may not be able to make a confident diagnosis. In many cases, they will have to request a repeat scan, delaying critical medical decisions and prolonging the time it takes for patients to receive treatment;
- increased Healthcare Costs: MRI machines are expensive, and their availability is often limited. If a scan needs to be repeated due to motion, it wastes valuable machine time, meaning fewer patients can be scanned in a day. This leads to higher operational costs for hospitals and can contribute to longer wait times for other patients;
- problem Discovered Too Late (A Posteriori Issue): One of the most frustrating aspects of bulk-motion is that it is often only discovered after the scan is completed. Unlike some imaging techniques where issues can be corrected in real-time, MRI scans typically need to be fully processed before a radiologist can assess the quality. If motion artifacts are found at this stage, the patient may have already left, requiring them to return for another scan, adding inconvenience and further delays;
- more Stress for Patients: MRI scans can already be uncomfortable, especially for people who are anxious, claustrophobic, or in pain. If a patient is told that their scan needs to be redone due to motion, it can lead to frustration, anxiety, and discomfort.

It is therefore desired to find a solution for real-time bulk-motion detection, capable of overcoming the previously mentioned disadvantages.

### Summary of Invention

An objective of the present invention is to propose a method and a system for real-time bulk-motion detection of a biological object during MRI of an internal part of said biological object that is characterized by a repeated motion, i.e. a motion that repeats over time, e.g. a periodical physiological motion.

This objective is achieved by the measures taken in accordance with the independent claims. Further advantageous embodiments are proposed by the dependent claims.

More precisely, the present invention concerns a method for real-time bulk-motion detection of a biological object during MRI of an internal part of said biological object, wherein said internal part is characterized by a repeated motion over time (e.g. a periodic (physiological) motion), said method comprising:
- launching an acquisition stage, wherein during said acquisition stage an MRI signal that enables a 2D or 3D image reconstruction of said internal part and, a tracker signal modulated by, or encoding, said repeated motion of said internal part of the biological object are acquired;
characterized in that it comprises:
- using the tracker signal acquired during a reference stage for automatically extracting or determining, from said tracker signal acquired during said reference stage, a reference pattern or model of said repeated motion, wherein said reference stage is comprised within said acquisition stage, takes place at the beginning of said acquisition stage, and extends over a time period T_{ref} covering at least one repetition of said repeated motion;
- after the end of said time period T_{ref}, i.e. once the reference stage is completed, and in particular during a monitoring stage of said acquisition stage, wherein the monitoring stage extends over a time period Tₘₒₙ, with notably T_{ref} + Tₘₒₙ = T_{ac}, using said reference pattern (or model) for automatically detecting, in real time and from the currently acquired tracker signal, a bulk-motion of the biological object; and
- outputting an alert in function of the detected bulk-motion.

The present invention concerns also a system for detecting in real-time bulk-motion of a biological object during an acquisition of images of an internal part of said biological object, wherein said internal part is characterized by a repeated motion over time, said system comprising:
- an interface for acquiring, notably from an imaging module configured for imaging the biological object, an MRI signal that comprises MRI data enabling an image reconstruction of at least said internal part of the biological object and a tracker signal modulated by said repeated motion of said internal part of the biological object;
- a control unit configured for processing the acquired MRI signal and tracker signal;
- an output interface configured for outputting an alert in function of a detected bulk-motion of said biological object;
the system being characterized in that it is configured to carry out the steps of the previously described method.

### Description of Embodiments

According to the present invention, the acquisition stage comprises two "sub" stages, which are the so-called reference stage and said monitoring stage, wherein a duration of the acquisition stage extends preferentially over a time period T_{ac}, with T_{ref} < T_{ac}. During said acquisition stage, the MRI signal and tracker signal are acquired, notably synchronously, by the system according to the invention, for instance an MRI apparatus. The tracker signal may comprise a repeated (e.g. periodical) change over time that represents or encodes said repeated motion over time. Said MRI signal comprises typically MRI data acquired during successive MRI readout blocks which are used for reconstructing said 2D or 3D image of at least said internal part of the biological object. Preferentially, the MRI signal and the tracker signal run in parallel. Preferentially, they are acquired at the same time (for instance, they are interleaved signals) by the system, so that an event, like a bulk-motion of the biological object, that takes place at a time Tₑᵥₑₙₜ might be identified in both signals in signal data acquired at Tₑᵥₑₙₜ. According to the present invention, the reference pattern or model describes or characterizes the repeated motion of the internal part. In particular, said reference pattern or model comprises reference motion parameters describing or encoding a motion behavior of said internal part.

Preferentially, the acquired tracker signal is represented by, or as, or comprises a series of temporally successive datapoints, wherein each datapoint is associated to a different one of MRI readout blocks used for acquiring the MRI signal, wherein each datapoint is constructed from data of the tracker signal that have been acquired during a time interval that ends at the end or at the start of the MRI readout block to which it is associated and that does not comprise any other MRI readout block performed by the MRI apparatus. The MRI readout blocks are configured for acquiring MRI data used for said reconstruction of the image of the biological object. Preferentially, each datapoint is further assigned to an acquisition time (each datapoint being assigned to a different acquisition time), wherein the acquisition time assigned to a datapoint is a time belonging to said time interval and that has been selected for representing said time interval during which said tracker signal data have been acquired. It can be for instance the starting time of said time interval, or the starting time of the readout block to which the datapoint is associated to. According to the present invention, each datapoint is preferentially a multidimensional data or vector.

According to the present invention, the control unit is preferably configured for using a dimensionality reduction technique for processing said tracker signal, and the reference pattern or model is defined in a dimensionality-reduced space. In particular, for extracting or determining said reference pattern, the control unit may apply a dimensionality reduction technique to the datapoints acquired during the reference stage - called hereafter the reference datapoints - for transforming said reference datapoints into dimensionality-reduced reference datapoints in a dimensionality-reduced space, wherein said reference pattern is defined in said dimensionality-reduced space from the dimensionality-reduced reference datapoints, and wherein, after completion of said reference stage, newly acquired datapoints of the tracker signal are projected onto said dimensionality-reduced space for determining, for each newly acquired datapoint, whether it matches with said reference pattern or not, and if not, then triggering said alert.

A preferred dimensionality reduction technique is a Principal Components Analysis, also called PCA, which is a linear dimensionality reduction technique. However, a non-linear dimensionality reduction technique might also be contemplated. Typically, the control unit is configured for applying said dimensionality reduction technique to each datapoint constructed from the tracker signal. For instance, the control unit may apply a PCA onto the reference datapoints for transforming each reference datapoint into its corresponding dimensionality-reduced reference datapoint, wherein said dimensionality-reduced space is characterized by N principal components (with preferentially N < 5 - as known in the art, the dimensionality-reduced space is characterized by a new coordinate system defined by the principal components of the PCA). In particular, the reference pattern might be then defined in function of a reference cluster Cluster_{ref} determined by the control unit by performing a cluster analysis on said dimensionality-reduced reference datapoints. As a result of said cluster analysis, Cluster_{ref} may comprise for instance all said dimensionality-reduced reference datapoints or a majority of them obtained via a statistical selection. Preferably, determining if a newly acquired datapoint matches with said reference pattern comprises determining whether the Eigenvalue of a dimensionality-reduced datapoint corresponding to said newly acquired datapoint is within a predefined distance from Cluster_{ref}, notably from a center of said reference cluster Cluster_{ref}, in said dimensionality-reduced space. In particular, said predefined distance might be defined in function of a standard deviation σ calculated from the Eigenvalues of the dimensionality reduced reference datapoints.

In a preferred embodiment, said reference pattern might be defined as a boundary automatically calculated by the control unit for enclosing a region comprising all or a majority of the dimensionality-reduced reference datapoints of Cluster_{ref}, said boundary being defined for instance as a set of points within said dimensionality-reduced space that are located at a distance equal to 2σ (in this case, the predefined distance equal 2σ) from said center of Cluster_{ref}, wherein said center might be defined as the mean value of the Eigenvalues of the dimensionality-reduced reference datapoints. In particular, after completion of the reference stage, i.e. during the monitoring stage, each datapoint for which its corresponding dimensionality-reduced datapoint is within said region is considered as free of bulk-motion, while a dimensionality-reduced datapoint being outside of said region is considered as subject to bulk-motion. Preferentially, the alert is not triggered as soon as a single datapoint is identified as being subject to bulk-motion, but only after a certain number of such datapoints are considered as being subject to bulk-motion. For instance, said alert might be triggered after a ratio of a number of dimensionality-reduced datapoints falling outside said region and a total number of datapoints that is expected to be acquired during said acquisition stage exceeds a predefined threshold (e.g. defined as a percentage, like 10%).

Preferentially, the tracker signal according to the invention might be an MRI signal configured for providing a 1D projection image in the SI direction (i.e. a SI profile) of said internal part of the biological object and that is acquired before each MRI readout block. In this case, each temporally successive datapoint is a SI profile constructed from an appropriate MRI signal acquired before an MRI readout block it directly precedes, comprising thus MRI data sampled from a given number of MRI coils. Alternatively, said tracker signal might be a 2D or 3D MRI navigator signal. In this case, each temporally successive datapoint is typically constructed from the navigator signal acquired before an MRI readout block it directly precedes. According to another alternative, a Pilot tone signal might be used as tracker signal. In this case, each temporally successive datapoint is typically constructed from (or comprises) PT signal data acquired during a time interval separating two directly successive MRI readout blocks. Other types of tracker signals might be contemplated, as long as said tracker signal is modulated by the repeated motion of the internal part of the biological object.

Preferentially, once an alert has been triggered, i.e in case of a detection of bulk-motion, the control unit may automatically restart the monitoring stage or may repeat the MRI readout block for which bulk-motion has been detected.

### Brief Description of the Drawing

Further aspects of the present invention will be better understood through the following drawings, wherein like numbers designate like objects:
- Fig. 1: schematic illustration of a system according to the invention;
- Fig. 2: flowchart of a preferred embodiment of a method according to the invention;
- Fig. 3: example of an imaging signal according to the invention;
- Fig. 4: illustration of a reference pattern according to the invention;
- Fig. 5: illustration of a detection of bulk-motion of a biological object based on MRI SI-profiles;
- Fig. 6: illustration of MRI readout blocks and datapoints according to the invention.

### Description of Examples

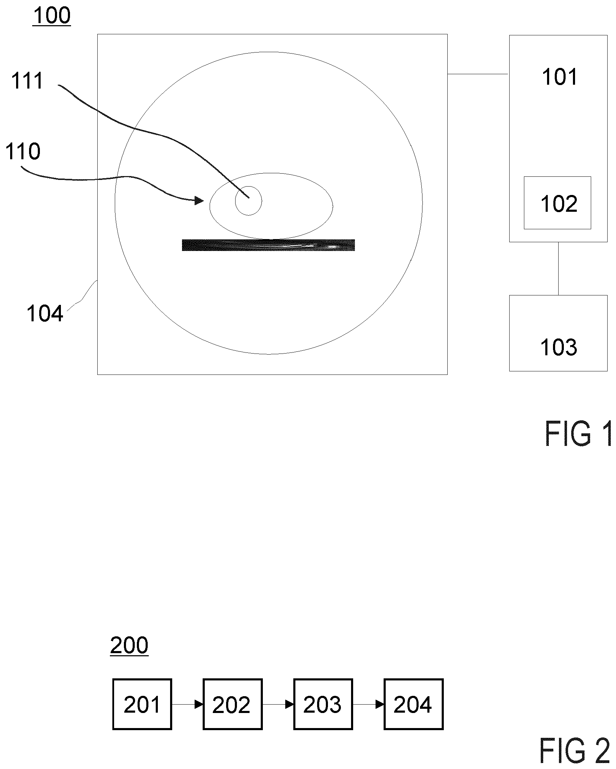

Figure 1 illustrates very schematically a system 100, e.g. an MRI apparatus, according to the invention. Said system 100 is configured for detecting in real-time bulk-motion of a biological object 110 during an acquisition of images of an internal part 111 of said biological object 110, wherein said internal part 111 is a part that has a repeated (e.g. periodic or quasi-periodic) physiological motion over time (e.g. respiratory motion of an organ or of part of a biological object over time caused by breathing). Said internal part 111 might be for instance an organ characterized by a repeated motion, like the heart or lung, or a tissue (i.e. a region of the biological object) driven into repeated motion by the motion (e.g. by the heartbeat) of an organ that is itself characterized by a physiological repeated/periodic motion (e.g. repeated cycles of inspiration and expiration of an organ or a body part in connection with breathing). The system 100 comprises:
- an interface for acquiring an MRI signal that comprises imaging data (i.e. MRI data) enabling a 2D or 3D image reconstruction of at least said internal part 111 of the biological object 110 and a tracker signal modulated by said repeated motion of said internal part 111 of the biological object 110. Said MRI signal might be acquired by said interface from an imaging module 104, e.g. an MRI scanner of the MRI apparatus, configured for imaging the biological object 210. Preferentially, said MRI signal and said tracker signal are both acquired (i.e. their corresponding raw data are acquired) by the imaging module 104;
- a control unit 101, typically comprising a processor and a memory 102, said control unit 101 being configured for processing the acquired MRI signal and tracker signal. The control unit 101 might be also configured for controlling the imaging module 104 for performing said acquisition of the MRI signal and of the tracker signal;
- one or several output interfaces 103 configured for outputting an alert in case of a detection of a bulk-motion of the biological object 110 from said tracker signal during imaging of the biological object 110. Said output interfaces may comprise for instance a display and/or a loudspeaker and/or a vibration device for alerting the operator about a detected bulk-motion via respectively a visual signal displayed on said display and/or an audible signal outputted by the loudspeaker and/or a vibration generated by the vibration device.

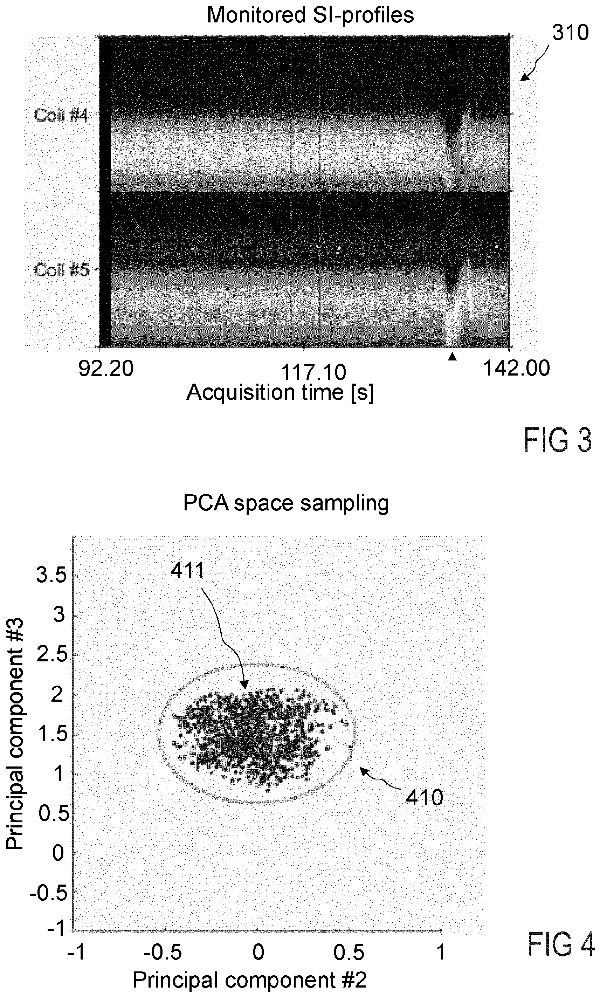

In connection with Figure 2, the principles of the method 200 for real-time bulk-motion detection will be described in more detail hereafter together with the examples of Figures 3 to 6. In particular, the method 200 according to the invention will be illustrated by means of a specific example based on a free-running whole-heart MRI. However, the concept of the present invention is not limited to this specific example, and may apply to other MRI image acquisition techniques of a biological object 110 based for instance on different types of tracker signals.

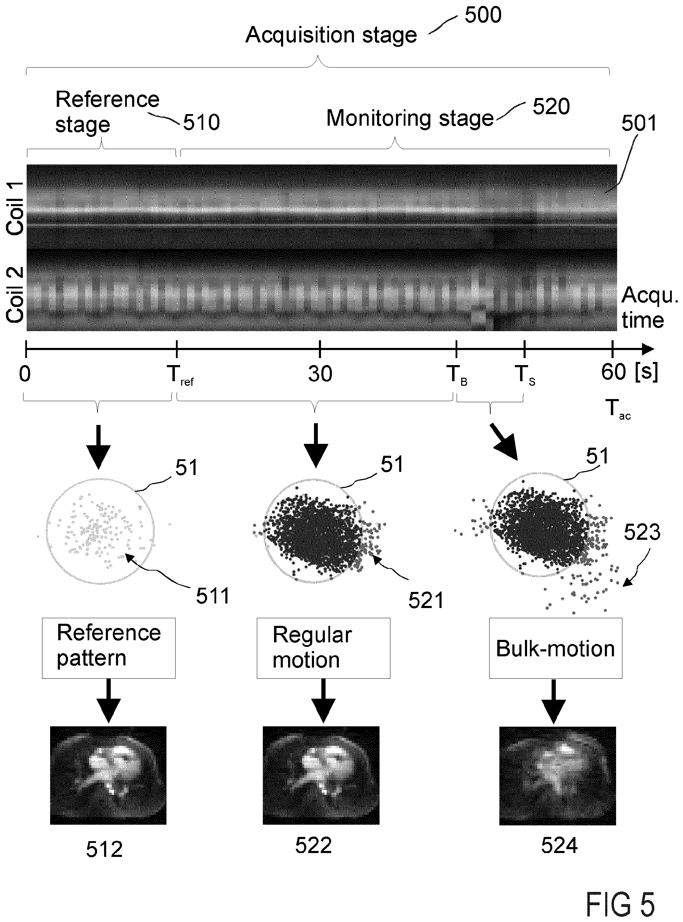

At step 201, the system 100 launches an acquisition stage 500 for acquiring MRI images of said biological object 110. According to the present invention, said acquisition stage 500 comprises a reference stage 510 and a monitoring stage 520, as illustrated in Figure 5. The reference stage 510 temporally takes place before the monitoring stage 520. For instance, the system 100 automatically starts the reference stage 510 when launching the acquisition stage 500. In other words, the acquisition stage 500 may directly start with the reference stage 510, and the monitoring stage 520 automatically starts once the reference stage 510 is completed. Typically, if the duration (i.e. the period of time over which the concerned stage extends) of the acquisition stage 500 is T_{ac}, the duration of the reference stage T_{ref}, and the duration of the monitoring stage Tₘₒₙ, then T_{ref} + Tₘₒₙ = T_{ac}. In case of a detection of bulk-motion, the system 100 may automatically stop the monitoring stage before the end of the acquisition stage, in which case T_{ref} + Tₘₒₙ < T_{ac}. In any case, T_{ref} is far much smaller than T_{ac}, i.e. T_{ref} < T_{ac} (e.g. 2s <T_{ref}<10s while 60s<T_{ac}<600s). An advantage of having a temporally short T_{ref} is a decrease of the likelihood of having a bulk-motion taking place during T_{ref}, i.e. during the acquisition of the tracker signal.

During said acquisition stage 500, the system 100 according to the invention acquires or receives, notably via its interface, at least the two following signals: the so-called tracker signal and said MRI signal. The MRI signal is used for imaging the internal part 111 of the biological object 110, while the tracker signal is used for tracking bulk-motion of said biological object 110. The tracker signal and the MRI signal might be acquired in parallel, or might be interleaved. At least during the monitoring stage 520, preferentially during the whole acquisition stage, said signals are acquired synchronously, i.e. they share a common time scale which enables to correlate bulk-motion events detected from the tracker signal with MRI data of the MRI signal. An example of MRI signal according to the invention is a free-running whole-heart MRI signal. However, any MRI signal that can be synchronously acquired with a tracker signal according to the invention might be used. The MRI signal comprises MRI data used, notably by the system 100 according to the invention, for reconstructing a 2D or 3D view of the biological object 110 comprising the internal part 111. For instance, the free-running whole-heart MRI signal is typically used for cardiac and respiratory motion-resolved imaging, wherein, unlike traditional MRI techniques that require careful coordination and patient cooperation (such as breath holding), a continuous acquisition of the MRI signal is performed free of any need to pause breathing for the patient undergoing said MRI.

More precisely, the MRI signal used for free-running whole-heart MRI typically captures 3D images of the heart in motion by collecting data in small time intervals, called "shots". Each shot is an MRI readout block configured for acquiring MRI data enabling a 3D image reconstruction of the biological object, for instance the heart and its surrounding tissues. Unfortunately, as in all common thoracic and abdominal MRI techniques, bulk-motion is also not accounted for in known in the art free-running whole-heart MRI techniques, which can lead to image artifacts limiting diagnostic image quality. The negative effects of bulk-motion are often discovered a posteriori, potentially forcing an exam to be repeated. The present invention solves this issue by automatically determining in real time if bulk-motion occurs from said tracker signal.

In particular, the present invention proposes to use Sl-profiles as tracker signal for detecting bulk-motion in real time, notably during free-running whole-heart MRI. For each shot, preferentially right before the concerned shot, MRI data (i.e. data acquired via the coils of the MRI scanner) enabling a computing of an Sl-profile are acquired, wherein said Sl-profile is an 1D integral of the biological object along the Sl-direction that is computed for one or several MRI acquisition coils. Such Sl-profiles 310, 501 are illustrated in Figures 3 and 5, wherein in Figure 3, the Sl-profiles in function of the acquisition time are shown for (central chest) coil #4 and coil #5 of the MRI apparatus, while in Figure 5, Sl-profiles for (central chest) coils #1 and #2 are presented. In the example of Figure 3, cough occurred at an acquisition time of 135 s (see the arrow), which generated bulk-motion detected via the tracker signal in real time. According to the present invention, each Sl-profile (e.g. the Sl-profile obtained at a given acquisition time for coils 1 and 2 in Figure 5) is a so-called datapoint of the tracker signal. Preferentially, for each shot, an Sl-profile (i.e. datapoint) is recorded and associated to the shot. The Sl-profiles 310, 501 are notably used as a measurement of how the heart and diaphragm move up and down during breathing, being therefore modulated by the repeated motion of the internal part (heart) of the biological object (a patient). While in the examples illustrated in Fig. 3 and 5, said tracker signal is represented by Sl-profiles 310, 501 acquired or monitored by the system 100 over the time of acquisition, the tracker signal is not limited to Sl-profiles. Indeed, a tracker signal according to the invention is any signal that is modulated by the repeated motion of the internal part 111 of the biological object 110 and that can be acquired synchronously with the MR imaging signal. Preferentially, raw signals corresponding to said tracker signal and said MR imaging signal are acquired by one and the same device, e.g. an MRI scanner (i.e. its coils). This preferentially ensures synchronicity between the tracker signal and the MRI signal.

According to the present invention, the tracker signal can be for instance a Pilot Tone signal, or 2D or 3D MRI navigator signal, etc.

At step 202, in order to enables a detection of bulk-motion in real time, the system 100, notably its control unit 101, uses the tracker signal acquired during the reference stage 510 for automatically extracting or determining, from said tracker signal, a reference pattern or model of said repeated motion. A reference pattern 410 according to the invention is illustrated in Figure 4 in connection with Sl-profiles and will be explained in more detail hereafter. The reference pattern 410 or model describes or characterizes the repeated motion of said internal part 111. In particular, said reference pattern or model may comprise, or might be defined by, reference motion parameters (or features) capable of describing or encoding the motion behavior of said internal part 111 in their respective values. The reference stage 510 is thus used by the system 100 according to the present invention for determining said reference pattern.

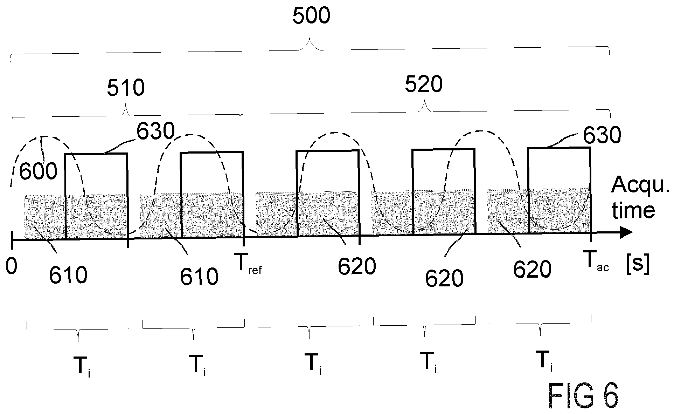

As very schematically illustrated in Figure 6, the tracker signal that is acquired by the system according to the invention might be represented as, or by, or comprises, a series of temporally successive datapoints, wherein a first series of said temporally successive datapoints is acquired during the reference stage 510 of the acquisition stage 500, and will be referred to hereafter as "reference" datapoints 610, to distinguish them from the datapoints 620 acquired during the monitoring stage 520 and which form a second series of temporally successive datapoints. Preferentially, the system 100 automatically defines the temporal duration T_{ref} of the reference stage 510 for ensuring that the latter covers at least one repetition of the repeated motion of the internal part 111 of the biological object 110. In Fig. 6, said repeated (periodic) motion is schematically illustrated by the dotted line 600. Preferentially, said duration T_{ref} is also defined for ensuring that the system 100 acquires a sufficiently high number of reference datapoints 610 for enabling a characterization of the repeated motion by the reference pattern. Typically, the number of reference datapoints acquired during the reference stage 510 is greater than 20. For illustration, Fig. 6 shows only two reference datapoints for the sake of clarity and ease of understanding. The series of rectangles 630 represents temporally successive MRI readout blocks that are configured for acquiring the MRI signal used for reconstructing a 2D or 3D image of the internal part 111 of said biological object. Whatever the datapoint (i.e. independently from the fact it is a reference datapoint 610 or a datapoint 620 acquired during the monitoring stage 520), each datapoint 610, 620 is associated to a different one of said MRI readout blocks, wherein each datapoint is constructed from data of the tracker signal that have been acquired during a time interval that ends at the starting time, or preferentially at the ending time, of the MRI readout block 630 that is associated to said datapoint, wherein said time interval does not comprise any other MRI readout block 630 (i.e. during said time interval no other MRI readout block may take place - for instance, it does not comprise the ending time of another MRI readout block). Said time interval Tᵢ is represented by the light gray rectangles in Fig. 6. Each datapoint according to the invention is thus constructed from the data of the tracker signal that are picked up in said time interval Tᵢ which comprises the interval of time during which the MRI readout block to which the datapoint is associated to is performed, and only the performing of this MRI readout block, preferentially ending at the ending time of the performing of said MRI readout block, starting before the starting time of said MRI readout block (but free of any performing of another MRI readout block). Preferentially, each datapoint is assigned an acquisition time, which can be for instance chosen within said time interval Tᵢ, for instance it can be the time at which the MRI readout block to which it is associated starts (i.e. said starting time). Said acquisition time enables to link the datapoint to the time interval during which said tracker signal data have been acquired for constructing the datapoint. For each datapoint, its associated acquisition time corresponds to a time value that belongs to said time interval Tᵢ. According to the present invention, each datapoint is a multidimensional data or vector. For instance, if the tracker signal is an MRI signal configured for providing a 1D projection image in the SI direction (i.e. a SI profile) of said internal part of the biological object and that is acquired before each MRI readout block, then each temporally successive datapoint is a SI profile constructed from an (appropriate) MRI signal acquired before the MRI readout block it is associated to, comprising thus MRI data sampled from a given number of MRI coils. In Figure 3 and 5, each Sl-profile is a datapoint according to the invention. If the tracker signal is a PT signal, each temporally successive datapoint is typically constructed from or comprises PT signal data acquired by one or several MRI coils during said time interval Tᵢ. If the tracker signal is a 2D or 3D MRI navigator signal, then each temporally successive datapoint might be typically constructed from the navigator signal acquired before the MRI readout block it is associated to. According to the present invention, each datapoint comprises thus a series of data of the tracker signal and is constructed for being represented as a multidimensional data or vector.

Indeed, in order to enable a real time detection of bulk-motion, the present invention proposes to use a dimensionality reduction technique for processing said datapoints and extracting or determining the reference pattern (or model). In "real time" means that for each MRI readout block (e.g. each shot), the system 100 (via its control unit 101) evaluates (or detects) whether a bulk-motion occurred with respect to said MRI readout block, and the result of said evaluation (or detection) is obtained before the next MRI readout block takes place, i.e. before it is performed by the MRI apparatus. Applying said dimensionality reduction to each datapoint acquired during the monitoring phase and using said reference pattern enable to speed up the detection of bulk-motion, making real time detection possible. The result regarding the occurrence or not of a bulk-motion is obtained thus in real time with respect to the acquisition of the MRI signal, which enables to take decision (e.g. stopping the monitoring stage, or repeating a shot, etc.) at a very early stage.

During the reference stage, the system 100, e.g. its control module 101, is configured for extracting or determining said reference pattern by applying a (linear like PCA or non-linear) dimensionality reduction technique to the reference datapoints for transforming said reference datapoints into dimensionality-reduced reference datapoints in a dimensionality-reduced space. This is illustrated in Figure 4 by the PCA space, wherein each dot has been obtained by applying to the Sl-profiles of Figure 3 a PCA, each dot representing thus a dimensionality-reduced datapoint (that corresponds to an Sl-profile) in a two-dimensional PCA space, the latter being defined by its second and third principal components. In said dimensionality-reduced space, each dimensionality-reduced reference datapoint is represented as a "dot" whose position within said dimensionality-reduced space is defined by its Eigenvalues.

The reference pattern is determined or constructed in said dimensionality-reduced space from the dimensionality-reduced reference datapoints. In particular, the dimensionality-reduced reference datapoints typically form a cluster, called reference cluster Cluster_{ref} 411, 511, that is used for defining or determining said reference pattern as illustrated in Figure 4 and 5. For instance, the control unit 101 may perform a cluster analysis on said dimensionality-reduced reference datapoints for determining said reference cluster Cluster_{ref} 411, 511 and may define or extract said reference pattern from Cluster_{ref} 411, 511. For instance, said reference pattern 410, 510 might be a boundary that is automatically calculated by the control unit 101 and that is configured for enclosing a region within the dimensionality-reduced space that comprises all or a majority of the dimensionality-reduced reference datapoints of Cluster_{ref}. An elliptical boundary is for instance illustrated in Figure 4, and a circular boundary (see ref. 51) is illustrated in Figure 5. In particular, said boundary might be defined in function of the center of Cluster_{ref} and a standard deviation σ calculated from the Eigenvalues of the dimensionality reduced reference datapoints. Said center and said standard deviation are for instance reference motion parameters according to the invention. In particular, the radius of the circular boundary 51 illustrated in Figure 5 might be equal to 2σ, said circular boundary being centered on the mean value of the Eigenvalues of the dimensionality-reduced reference datapoints. At the end of the reference stage 510, the system 100 may store said reference pattern 410, 51 in its memory. During the reference stage 510, a reference image 512 of the biological object 110 might also be acquired.

At step 203, after the end of said time period T_{ref}, i.e. once the reference stage is completed, the reference pattern 410, 51 is used by the control unit 101 for automatically detecting, in real time and from the currently acquired tracker signal, whether a bulk-motion of the biological object 110 occurs or not. This monitoring of bulk-motion takes place during the so-called monitoring stage 520. Typically, during the monitoring stage 520, the control unit 101 continuously constructs from the tracker signal datapoints, one after the other, and projects each of said newly constructed datapoint onto the dimensionality-reduced space (determined for and from the dimensionality-reduced reference datapoints) for determining, for each newly constructed datapoint, whether it matches with said reference pattern or not, and if not, then the control unit 101may trigger an alert. As illustrated in Figure 5, newly constructed datapoints result in new dimensionality-reduced datapoints, wherein some are located within the circular boundary 51, and other 521 are located outside said circular boundary 51. Typically, if the Eigenvalue of a new dimensionality-reduced datapoint is within a predefined distance from the center of Cluster_{ref} in the dimensionality-reduced space, then the control unit 101 is configured for labelling said new dimensionality-reduced datapoint as being free of any bulk-motion, otherwise, if it is not within said predefined distance, then said new dimensionality-reduced datapoint is labelled as affected by bulk-motion. Said predefined distance may correspond for instance to the radius of the circular boundary 51 illustrated in Figure 5. During the monitoring stage 520, images 522 of the biological object are reconstructed and preferentially stored as long as no alert for bulk-motion is generated by the system 100.

At step 204, the system 100 according to the invention, notably its control unit 101, is configured for outputting an alert in function of the detected bulk-motion. Said alert might be outputted via said one or several output interfaces 103. Preferentially, said alert is triggered after a ratio of a number of dimensionality-reduced datapoints that are not within said predefined distance from Cluster_{ref} and a total number of datapoints that is expected to be acquired during said acquisition stage exceeds a predefined threshold (e.g. defined as a percentage, like 10%). This is illustrated in Figure 5, wherein until the acquisition time T_{B}, while new dimensionality-reduced datapoints 521 were located outside the circular boundary 51, no alert was triggered because said ratio (which is continuously updated (i.e. updated over time) by the system 100 in function of each newly calculated dimensionality-reduced datapoint) did not yet exceed said predefined threshold. However, during the laps of time separating T_{B} from T_{S}, additional dimensionality-reduced datapoints 523 fall outside the reference pattern (i.e. said circular boundary 51), and, at time T_{S}, the predefined threshold is finally exceeded, and an alert is automatically generated by the system 100. For illustration purpose, the image 524 acquired during said bulk-motion shows a decreased quality compared to the image 522 acquired during regular motion, i.e. as long as said ratio was not exceeded. As explained earlier, said ratio evolves or changes over time during the monitoring stage 520, being re-evaluated each time a new dimensionality-reduced datapoint is calculated.

To conclude, the present invention proposes a method and a system for real-time bulk-motion detection which use dimensionality reduction for building a (case-specific) reference pattern or model for bulk-motion based on a tracker signal that is typically acquired by an MRI scanner. Advantageously, the proposed invention can be deployed to detect bulk-motion with minimal delays during data acquisition. Additionally, the reference pattern or model is built specifically for the biological object under investigation at the beginning of the acquisition stage, being therefore specifically tuned for said biological object and providing a more robust and accurate bulk-motion detection, increasing the efficiency of MRI scanning and mitigating motion distortions.

## Claims

1. Method (200) for real-time bulk-motion detection of a biological object (110) during magnetic resonance imaging, hereafter MRI, of an internal part (111) of said biological object (110), wherein said internal part (111) is **characterized by** a repeated motion over time, said method (200) comprising:
- launching (201) an acquisition stage (500), wherein during said acquisition stage (500), an MRI signal that enables an image reconstruction of said internal part (111) and a tracker signal modulated by said repeated motion of said internal part (111) of the biological object (110) are acquired;
**characterized in that** the method comprises
- using (202) the tracker signal acquired during a reference stage (510) for automatically extracting or determining, from said tracker signal, a reference pattern (410) of said repeated motion, wherein said reference stage (510) is comprised within said acquisition stage (500), takes place at the beginning of said acquisition stage (500), and extends over a time period T_{ref} covering at least one repetition of said repeated motion;
- after the end of said time period T_{ref}, using (203) said reference pattern (410) for automatically detecting, in real time and from the currently acquired tracker signal, a bulk-motion of the biological object (110); and
- outputting (204) an alert in function of the detected bulk-motion.

2. Method (200) according to claim 1, wherein the acquired tracker signal is represented by, or comprises, a series of temporally successive datapoints, wherein each datapoint is associated to a different one of MRI readout blocks used for acquiring the MRI signal, wherein each datapoint is constructed from data of the tracker signal that have been acquired during a time interval that ends at the end or at the start of the MRI readout block to which it is associated and that does not comprise any other MRI readout block, each datapoint being a multidimensional data.

3. Method (200) according to claim 2, wherein, for extracting or determining said reference pattern (410), a dimensionality reduction technique is applied to the datapoints acquired during the reference stage - called hereafter the reference datapoints - for transforming said reference datapoints into dimensionality-reduced reference datapoints in a dimensionality-reduced space, wherein said reference pattern is defined in said dimensionality-reduced space from the dimensionality-reduced reference datapoints, and wherein, after completion of said reference stage, newly acquired datapoints of the tracker signal are projected onto said dimensionality-reduced space for determining, for each newly acquired datapoint, whether it matches with said reference pattern (410) or not, and if not, then triggering said alert.

4. Method (200) according to claim 3, wherein said dimensionality reduction technique is a PCA applied onto the reference datapoints for transforming each reference datapoint into its corresponding dimensionality-reduced reference datapoint, wherein said reference pattern (410) is defined in function of a reference cluster Cluster_{ref} determined by performing a cluster analysis on said dimensionality-reduced reference datapoints, wherein determining if a newly acquired datapoint matches with said reference pattern comprises determining whether the Eigenvalue of a dimensionality-reduced datapoint corresponding to said newly acquired datapoint is within a predefined distance from Cluster_{ref} in said dimensionality-reduced space.

5. Method (200) according to claim 4, wherein said predefined distance is defined in function of a standard deviation σ calculated from the Eigenvalues of the dimensionality reduced reference datapoints.

6. Method (200) according to claim 4 or 5, wherein said reference pattern (410) is defined as a boundary automatically calculated for enclosing a region comprising all or a majority of the dimensionality-reduced reference datapoints of Cluster_{ref}, and wherein, after completion of the reference stage (510), each datapoint for which its corresponding dimensionality-reduced datapoint is within said region is considered as free of bulk-motion, while a dimensionality-reduced datapoint being outside of said region is considered as subject to bulk-motion.

7. Method (200) according to claim 6, wherein said alert is triggered after a ratio of a number of dimensionality-reduced datapoints falling outside said region and a total number of datapoints that is expected to be acquired during said acquisition stage (500) exceeds a predefined threshold.

8. Method (200) according to claim 3, wherein said dimensionality reduction technique is a non-linear dimensionality reduction technique.

9. Method (200) according to one of the claims 2 to 8, wherein said tracker signal is:
- an MRI signal configured for providing a 1D projection image in the SI direction (i.e. a SI profile) of said internal part of the biological object and that is acquired before each MRI readout;
- 2D or 3D MRI navigator signal;
- a Pilot tone signal.

10. Method (200) according to one of the claims 1 to 9, comprising building or creating or extracting said reference pattern (410) in a dimensionality reduced space from data of the tracker signal acquired during the reference stage (510).

11. Method (200) according to one of the claims 1 to 10, wherein said acquisition stage (500) comprises the reference stage (510) and a monitoring stage (520) wherein said monitoring stage (520) is automatically restarted in case of a detection of bulk-motion.

12. System (100) for detecting in real-time bulk-motion of a biological object (110) during an acquisition of images of an internal part (111) of said biological object (110), wherein said internal part (111) is **characterized by** a repeated motion over time, the system (100) comprising :
- an interface for acquiring an MRI signal that comprises MRI data enabling an image reconstruction of at least said internal part (111) of the biological object (110) and a tracker signal modulated by said repeated motion of said internal part (111) of the biological object (110);
- a control unit (101) configured for processing the acquired MRI signal and tracker signal;
- an output interface (103) configured for outputting an alert in function of a detected bulk-motion of said biological object (110);
the system (100) being **characterized in that** it is configured to carry out the steps of the method according to one of the claims 1-11.

13. MRI apparatus comprising the system (100) according to claim 12, wherein said imaging module (104) is an MRI scanner, and the control unit (101) is configured for controlling the MRI scanner for performing said acquisition of the MRI signal and of said tracker signal.
